# EUROPEAN PATENT APPLICATION

(11) **EP 1 413 209 A1**
(43) Date of publication of application: **28.04.2004**
(21) Application number: 02751776.2
(22) Date of filing: 30.07.2002
(51) Int. Cl.: A23L 1/305

(54) **ERGOGENIC FOOD COMPOSITIONS**

(30) Priority: 31.07.2001 JP 2001230887
(71) Applicant: Ajinomoto Co., Inc., Tokyo 104-8315 (JP)
(72) Inventor: MAWATARI, Kazunori, Chuo-ku, Tokyo 104-8315 (JP); MURAKAMI, Hitoshi, Kawasaki-shi, Kanagawa 210-8681 (JP); SUZUKI, Hiromi, Kawasaki-shi, Kanagawa 210-8681 (JP)
(74) Representative: Strehl Schübel-Hopf & Partner
(86) International application number: PCT/JP2002/007701
(87) International publication number: WO 2003/011056

(57) **Abstract**

In this application are disclosed (a) a food composition for recovery from fatigue comprising aspartic acid as the active ingredient which composition has a recovery function from fatigue, and which composition is to be taken in an amount of 2 g or more in terms of said aspartic acid by an adult human per day, and (b) a food composition for recovery from fatigue comprising aspartic acid, and at least one other amino acid such as glutamic acid, as the active ingredient which composition has a recovery function from fatigue, and which composition is to be taken in an amount of 1 g or more in terms of said aspartic acid and in a total amount of 1 g or more in terms of said at least one other amino acid such as of glutamic acid, by an adult human per day. These food compositions allow an instant recovery from fatigue after an exercise or work requiring a physical strength and facilitate the training or the work the next day.

## Description

### (Technical Field)

The present invention relates to food compositions for recovery from fatigue such as a sports food/beverage for use by athletes, a nutritional supplementary food for those who feel fatigue in hard works in daily lives, and a nutrient or the like for recovery from fatigue.

### (Background Art)

As a sports food has heretofore been used a lot a nutritional supplementary food such as "Protein supplements" for increasing skeletal muscle mass and athletic performance. However, in actual fact, athletes suffer from an acute muscle fatigue or the like after exercise. It is supposed to be very significant for efficiently performing daily training not only to increase skeletal muscles but also to improve these various symptoms. It is also supposed to be very significant for those who suffer from acute tiredness after works requiring physical strength such as nuscular labor or the like to prevent the tiredness in advance or to improve once produced tiredness, in order to smoothly proceed with the work the next day.
For these purposes have been used nutritional supplementary foods containing "Protein supplements" or vitamins as major ingredients, or nutrients. However, with these, effects cannot actually be realized in many cases, and there has been a demand for development of more effective nutritional foods.
Additionally, a living body loses a large amount of energy during doing a hard exercise or work. It has heretofore been known that carbohydrate or fat is preferentially used as the energy source. On the other hand, protein is also used as the energy source at the time of the exercise in some cases, and, however, it has been known that protein is used as a constituent of skeletal muscles rather than as the energy source.

### (Disclosure of the Invention)

In the background of the background art described above, it is an object of the present invention to provide a method of instantly recovering from physical changes after an exercise or a work requiring a physical strength, that is, a method of instantly recovering from physical fatigue conditions to facilitate the training or the work the next day.

As a result of their intensive researches for achieving the object, the present inventors have found that quick recovery from fatigue conditions can be made by an intake of a certain amount or more of aspartic acid, especially its sodium salt, or at least one specific amino acid, in addition thereto, and have completed the present invention based on these findings.
Accordingly, the present invention relates to a food composition for recovery from fatigue comprising aspartic acid as the active ingredient which composition has a recovery function from fatigue, and which composition is to be taken in an amount of 2 g or more in terms of the aspartic acid by an adult human per day, as well as a food composition for recovery from fatigue comprising aspartic acid, and at least one amino acid selected from the group consisting of glutamic acid, glutamine, alanine, serine, glycine, threonine, cysteine, and proline as the active ingredient which composition has a recovery function from fatigue, and which composition is to be taken in an amount of 1 g or more in terms of the aspartic acid and in a total amount of 1 g or more in terms of said at least one amino acid selected from the group consisting of glutamic acid, glutamine, alanine, serine, glycine, threonine, cysteine, and proline, by an adult human per day, and further to the food compositions for recovery from fatigue, wherein the aspartic acid is especially in the form of a sodium salt.

Additionally, according to the researches by the present inventors (See the Experimental Examples described later), it has been indicated that several amino acids, especially glucogenic amino acids, are used in the living body more quickly than carbohydrates, whereby the ATP concentration is kept normal in the liver. Moreover, in accordance with the present inventors' researches, it has been recognized that, regarding the experimental animals loaded with an exercise for a long time, the ATP does not change in the skeletal muscles, but the ATP concentration instantly changes in the liver, and along this, the experimental animals are remarkably decreased in the amount of spontaneous activity.
According to the present invention, the glucogenic amino acids such as aspartic acid are remarkably effective in quickly recovering the liver ATP concentration and normalizing the spontaneous activity of animals in such case.
On the other hand, "Protein supplements" used a lot after the exercise is effective in increasing skeletal muscle mass, but the above-described recognized effects have not been reported therefor. These effects have not been recognized also in major ingredients of a nutrient such as taurine or the like frequently used for the recovery from fatigue.
Since a dosage of the aspartic acid contained in a commercial nutritional tonic beverage is small (a dosage at a time is 200 mg or less) , it is insufficient for the ATP amount in the liver to be recovered or the tiredness to be improved. Moreover, since taurine is insufficient in usability as an energy source in the living body, it does not sufficiently recover the ATP amount in the liver nor sufficiently improves the tiredness.

The present invention will hereinafter be described in detail.

Examples of the form of the food compositions for recovery from fatigue of the present invention include, in addition to soft drinks, from daily steady foods such as miniature drinks, jelly beverage, jelly, tablets, capsules, granulated powder, and tablet sweets or the like, to supplementary foods, nutrients, and the like. When a glucogenic amino acid is taken as a beverage, the acid is oxidized in a remarkably short time and used as an energy source.

Aspartic acid for use as the active ingredient of the food composition for recovery from fatigue is an amino acid which is not in the form of protein, different from an amino acid which is in the form of protein contained in a natural protein such as "Protein supplements" or the like. Aspartic acid can be used either in the L-form or the DL-form, but the L-form is preferable from the viewpoint of physiological usability.
As well known, aspartic acid is manufactured by a synthetic process, fermentative process, hydrolytic process of protein, and is usable in the food compositions of the present invention regardless of its source. Additionally, aspartic acid is, as such, preferably highly pure for physiological reasons, and it is preferable to use aspartic acid having a purity (98% or more) which is not less than that defined, for example, in "Japanese Standards of Food Additives".
Moreover, needless to say, aspartic acid may be in the form of a physiologically acceptable salt, and that in the form of a sodium salt is especially preferable among those in the salt forms. This is because the absorption of aspartic acid is sodium-dependent, and because aspartic acid is enhanced in solubilyty in the form of the sodium salt, though aspartic acid per se is inferior in solubility.
Furthermore, as for aspartic acid for use according to the present invention, it may be, or may be used, in the form of a peptide containing aspartic acid. As such peptides are preferable for the physiological reason those peptides wherein the aspartic acid is present in an amount of 30% or more of all the amino acids contained therein. These peptides may be either peptides manufactured by a synthetic process or peptides obtained by the hydrolysis of an animal or plant protein. As for these peptides, the molecular weight is preferably 2,000 or less, especially preferably 1, 000 or less, from the viewpoint of digestion and absorption. The ATP concentration in the liver can be quickly recovered, due to the fact that the peptide is instantly absorbed.
The other amino acids for use as the active ingredient of the food compositions for recovery from fatigue of the present invention, that is, glucogenic amino acids such as glutamic acid, glutamine, alanine, serine, glycine, threonine, cysteine, and proline may also be used in the form of a peptide containing the same.

The content of aspartic acid in the food compositions for recovery from fatigue of the present invention is preferably about 0.1 to 10%, more preferably about 0.5 to 2%, when they are in the form of liquid or jelly. This also applies, even when the food compositions are in the form of solid. When the food compositions are in the form of powder, the content of aspartic acid is preferably 1 to 20%, more preferably 2 to 10%.
Moreover, the food compositions for recovery from fatigue of the present invention can contain aspartic acid, and the glucogenic amino acids such as glutamic acid, glutamine, alanine, serine, glycine, threonine, cysteine, proline or the like, or a peptide containing these amino acids, in an amount occupying 30 to 100% of the food compositions.
Needless to say, aspartic acid may me used alone as the amino acid source. In a test in which aspartic acid was administered to mice, the results revealed that an active force enhancement function had been recognized with a dosage exceeding 2 g/kg (Experimental Example 4 described later).

When the dosage of an amino acid for human beings is to be estimated from the dosage for mice, the estimation is performed in the following way of thinking. That is, 'it can be supposed that a ratio of the amount of the protein taken per day by a mouse to the amount of the amino acids administered to the mouse for the purpose of adjustment of the physiological function and that of the amount of the protein taken per day by a human being to the amount of the amino acids administered to the human being for the purpose of adjustment of the physiological function are equal to each other. Rodents such as mice and rats take in a considerably large amount of feed per their weight as compared with human beings. Therefore, the metabolic amount by rodents of the amino acids is remarkably larger per their weight as compared with human beings, and, accordingly, the amount per weight, of the amino acids administered to mice or rats aiming at the physiological function adjustment is overwhelmingly large as compared with human beings. Therefore, the dosage is estimated in a way different from that of the dosage of general medicines (where extrapolation of the effective amount per weight as such to human beings from the experiment animals is made). As for a clinical nutrient (for a liver function improving medicine using branched-chain amino acids, or the like), as described above, supposing that the ratio of the amount of the protein taken per day by a mouse to the amount of the amino acids administered to the mouse for the above-described purpose and that for the human being are equal to each other, the human dosage is estimated from the result of the animal experiment.
In the case of the present invention, the effective amount for human beings is estimated from the experimental effective amount for the mice, that is, the protein amount per day for the mouse is about 0.9 g and an effective aspartic acid take-in amount per individual is 0.025 g, in the case where a minimum effective dosage of aspartic acid is 1 g/kg. When this ratio is multiplied by a protein take-in amount of 80 g per day of a human adult, the minimum effective dosage is 2 g or more per day for the human adult. In this manner, the human effective amount of aspartic acid was estimated. Therefore, a food composition containing aspartic acid in such an amount that the dosage of aspartic acid per day by an adult human can be 2 g or more, when taken in, is useful because this effective amount can efficiently be taken in, and moreover the effect of recovery from fatigue is also provided. The content (compounding ratio) of aspartic acid and the other glucogenic amino acids to be blended in a food composition for recovery from fatigue of the present invention is calculated back from an ordinary drink or eat amount per day depending upon the form of the food composition, and can also be determined as the compounding ratio satisfying a predetermined amount per day (2 g'or more of aspartic acid, or 1 g or more of aspartic acid and 1 g or more of another glucogenic amino acid).

In addition to aspartic acid or a peptide containing the same, and another glucogenic amino acid or a peptide containing the same, any one of usual blends into the food composition in this field, such as vitamin, carbohydrate, lipid, protein, vitamin, mineral, caffeine, herbal medicine, or the like, or a combination of two or more of such blends may also be blended with the food compositions for recovery from fatigue of the present invention. In this case, it is also possible to combine therewith a filler, a taste-curing agent, a dyestuff, or the like.

The food compositions for recovery from fatigue of the present invention manufactured in this manner can be put in distribution as such, that is, for example, in the form of a powder mixture, or in another appropriate form.

### (Brief Description of the Drawings)

Fig. 1 shows the measurement results with the passage of time, of the discharged ¹⁴CO₂ into the expired air of the ¹⁴C-labeled amino acid-administered mice (Experimental Example 1).
Fig. 2 shows the measurement results of the ATP/AMP ratios in the mouse livers (Experinental Example 2).
Fig. 3 shows the measurement results of the spontaneous activity of the mice after made to walk forcedly (Experimental Example 3).
Fig 4 shows the measurement results of the spontaneous activity of the mice after made to walk forcedly (Experimental Example 4).
Fig 5 shows the ATP/AMP ratio in the liver of the mice after restrained (administered 15 minutes before the end of the restraint, Experimental Example 5).
Fig 6 shows the ATP/AMP ratio in the liver of the mice after restrained (administered 30 minutes before the end of the restraint, Experimental Example 5).

### (Best Mode for Carring out the Invention)

In the following will be further described the present invention with reference to the experimental examples.

### <Experimental Example 1>

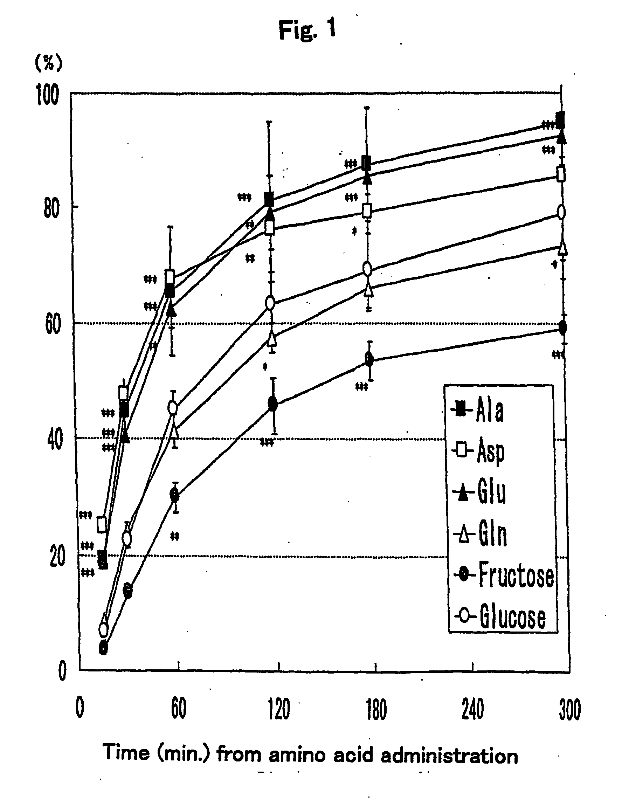

Each of L-Asp·Na·H₂O (Asp), L-Ala (Ala), L-Glu·Na·H₂O (Glu), L-Gln (Gln), D-fructose (Fructose) and D-glucose (Glucose) which were labeled with RI was injected (2 kBq/gBW) into the tail vein of each mouse of 6 groups of 6-week-old male CDF mice, each one group consisting of 5 mice (each group N = 5), and the animals were immediately placed in a metabolic cage. Thereafter, a specific radio-activity of the 20% monoethanolamine solution which had recovered ¹⁴CO₂ in the expired air or gas was measured at 5, 15, 30, 60, 120, 180, and 300 minutes. The results will be shown later in Fig. 1. In the drawing, each of *, **, *** indicates the level in the significant difference from the Glucose-administered group constituting the control group, and * indicates p < 0.05, ** indicates p < 0.01, and *** indicates p < 0.001.
As can be seen from Fig. 1, a discharged amount of the ¹⁴CO₂ into the expired air was increased at an earlier stage regarding each administered group of L-Asp, L-Ala, and L-Glu as compared with the D-glucose-administered group, and was at a higher level at every measured time. The discharged amount thereof into the expired air regarding the D-fructose-administered group was at lower values as compared with the D-glucose-administered group. The total discharged amount at 300 minutes after the administration of each of the L-Asp-, L-Ala-, and L-Glu-administered groups reached about 80% of the total administered radiation, indicating that these amino acids have a remarkably high usability in the living body. Moreover, the discharge of the ¹⁴CO₂ into the expired air immediately after the start of the administration was fastest regarding the L-Asp-administered group.

### <Experimental Example 2>

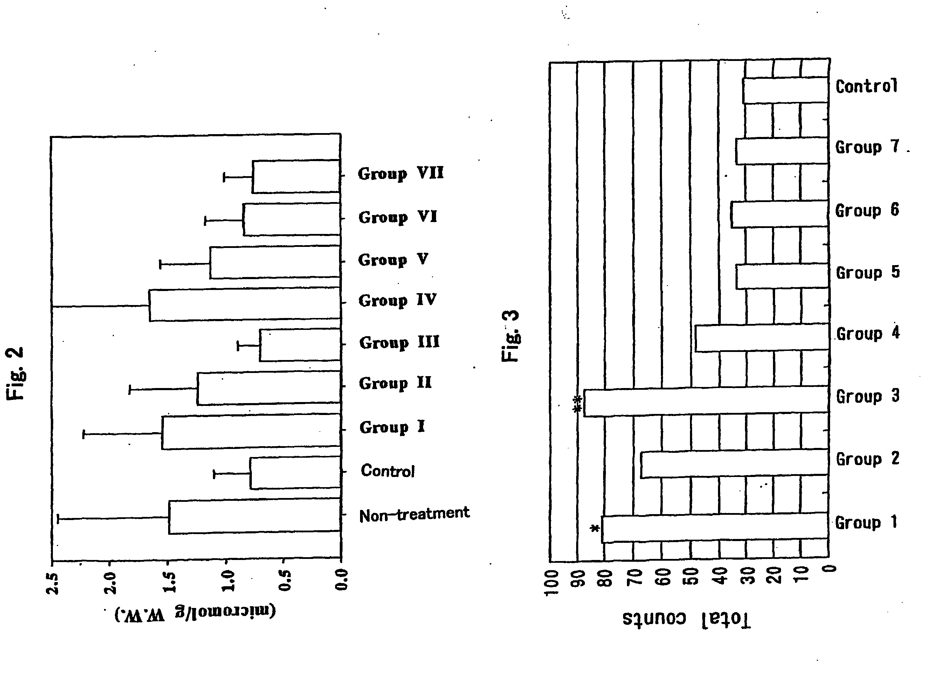

Seven groups (Groups I to VII) of 5-week-old male CDF1 mice, each one group consisting of 5 mice, were restrained for four hours, provided that ① L-Asp·Na·H₂O (Asp), ② a 1:1:1 mixture of Asp, L-Glu·Na·H₂O (Glu), and D-fructose (Fruc), ③ caffeine (Caf, 4 mg/kg (the same dosage for all the following groups), @ Asp+Caf, ⑤ a 1:1:1 mixture of Asp, Glu, and Fruc + Caf, ⑥ an 8:8:83 mixture of Asp, (L-Asp)₂Mg and taurine (Tau) +Caf, and ⑦ a 10: 82 : 8 mixture of Asp, Tau, and L-carnitine chloride (Car)+Caf were orally administered to the mice of Groups I to VII, respectively, 30 minutes before the end of the restraint in such an amount that the total weight of each amino acid(s) mixture or each amino acid(s) and sugar mixture was 2 g/kgBW. After the end of the restraint, the animals were killed, and the liver ATP/AMP ratio regarding each animal was measured immediately after death. For the sake of comparison, the liver ATP/AMP ratio was measured regarding of the mice of a group in which no restrained treatment was performed and no amino acid or sugar was administered (Non-treatment), and those of a control group in which the restrained treatment was carried out but no amino acid or sugar was administered. The results will be shown in Fig. 2 given later.
As can be seen from Fig. 2, the liver ATP/AMP ratios of the L-Asp-administered groups were higher than that of the caffeine (Caf)-administered group, and indicated a level higher than those of the compositions containing taurine as the major ingredient.

### <Experimental Example 3>

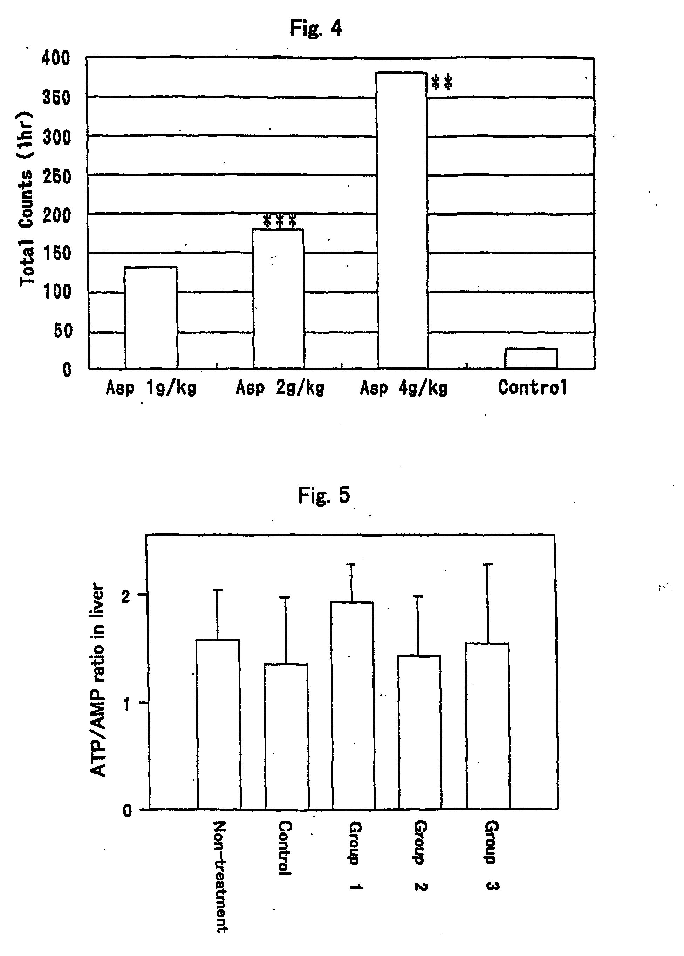

Seven groups (Groups 1 to 7) of 6-week-old male CDF1 mice, each one group consisting of 5 mice, were placed on a treadmill, and loaded with forced walking for three hours. Thereafter, (1) a glucogenic amino acid mixture of the asparagus composition (a 10:48:24:6:6:6 mixture of L-Ala, L-AspNa·H₂O, L-Glu·Na·H₂O (L-monosodium glutaminate 1 hydrate), Gly, L-Pro, and L-Ser, (2) L-Asp·Na·H₂O, (3) a 1:1:1 mixture of L-Asp·Na·H₂O, L-Glu· Na·H₂O, and fructose, (4) a 8:8:83 mixture of L-Asp·Na, (L-Asp)₂·Mg, and taurine, (5) a 10:82:8 mixture of L-Asp·Na·H₂O, taurine, and L-carnitine chloride, (6) taurine, and (7) a 91:9 mixture of taurine and L-carnitine chloride were orally administered to the mice of Groups 1 to 7, respectively, in such a manner that the total dosage of the amino acid(s) of each group was 2 g/kg (each, in the form of a 10% aqueous solution). Thereafter, the spontaneous activity was measured for 60 minutes, regarding the mice of the groups, with the use of a spontaneous activity measuring device with an infrared sensor, compared with that obtained regarding the mice of a control group (administered with only deionized distilled water). The results will be shown in Fig. 3 given later. In the figure, each of * and ** indicates the level in the significant difference from the control group, * indicates p < 0.05, and ** indicates p < 0.01.
As can be seen from Fig. 3, each of the group administered with the glucogenic amino acid mixture of the asparagus composition (Group 1), the group administered with only L-Asp. Na·H₂O (Group 2), and the group administered with the 1:1:1 mixture of L-Asp·Na·H₂O, L-Glu-Na·H₂O, and fructose (Group 3) was significantly improved in spontaneous activity, as compared with the control group. However, any effects were not recognized regarding the groups administered with the other amino acid compositions containing taurine as the major ingredient.

### <Experimental Example 4>

Three groups (Groups 1 to 3) of 6-week-old male CDF1 mice, each one group consisting of 5 mice, were placed on a tread mill, and loaded with forced walking for three hours. Thereafter, (1) 1 g/kg of L-Asp·Na·H₂O, (2) 2g/kg of L-AspNa·H₂O, and (3) 4g/kg of L-Asp·Na·H₂O were orally administered to the mice of Groups 1 to 3, respectively, (each, in the form of a 10% aqueous solution). Thereafter, the spontaneous activity was measured for 60 minutes, regarding the mice of the groups, with the use of a spontaneous activity measuring device with an infrared sensor, compared with that obtained regarding the mice of a control group (administered with only deionized distilled water). The results will be shown in Fig. 4 given later. In the figure, each of ** and *** indicates the level in the significant difference from the control group, ** indicates p < 0.01 and *** indicates p < 0.001.
As can be seen from Fig. 4, even the group administered with 1 g/kg of L-Asp·Na·H₂O (Group 1), was significantly improved in spontaneous activity, as compared with the control group. However, any effects were not recognized in experiments separately carried out from this, regarding groups administered with less than 1 g/kg of L-Asp·Na·H₂O.

### <Experimental Example 5>

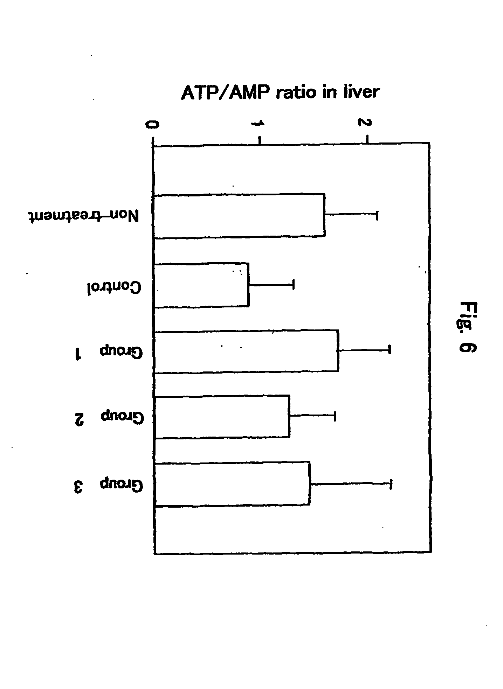

Three groups (Groups 1 to 3) of 5-week-old male CDF1 mice, each one group consisting of 5 mice, were restrained for three hours, using a restraining tube, provided that (1) L-Asp·Na· H₂O, (2) glucose, and (3) fructose were orally administered each in an amount of 2g/kg to the mice of the three groups, respectively, 15 minutes before the end of the restraint. The groups were then compared with (4) a group (Control) in which only an equal liquid volume of distilled water was administered, and (5) a non-treated group (Non-treatment). That is, each animal was killed immediately after the end of the restraint, and the ATP/AMP ratio in the liver was measured. The results will be shown in Fig. 5 given later.
As can be seen from Fig. 5, regarding the administration 15 minutes before the end of the restraint, the ATP/AMP ratio in the liver of the Asp-administered group (Group 1) indicated the highest level.
Even in the case of the administration 30 minutes before the end of the restraint, instead of the administration 15 minutes before the end of the restraint, a similar tendency was observed (see Fig. 6 given later).

### (Industrial Applicability)

According to the present invention, there can easily be provided food compositions for recovery from fatigue superior in the effect of the recovery from fatigue, whereby the energy metabolism of the liver is instantly improved and a vital force can more quickly be enhanced as compared with an ordinary nutrient containing amino acids such as "Protein supplements", taurine or the like as the major ingredient.

## Claims

1. A food composition for recovery from fatigue comprising aspartic acid as the active ingredient which composition has a recovery function from fatigue, and which composition is to be taken in an amount of 2 g or more in terms of said aspartic acid by an adult human per day.

2. The food composition for recovery from fatigue of Claim 1 which further comprises, in addition to said aspartic acid, at least one amino acid selected from the group consisting of glutamic acid, glutamine, alanine, serine, glycine, threonine, cysteine, and proline.

3. A food composition for recovery from fatigue comprising aspartic acid, and at least one amino acid selected from the group consisting of glutamic acid, glutamine, alanine, serine, glycine, threonine, cysteine, and proline as the active ingredient which composition has a recovery function from fatigue, and which composition is to be taken in an amount of 1 g or more in terms of said aspartic acid and in a total amount of 1 g or more in terms of said at least one amino acid selected from the group consisting of glutamic acid, glutamine, alanine, serine, glycine, threonine, cysteine, and proline, by an adult human per day.

4. The food composition for recovery from fatigue of any one of Claims 1-3, wherein said aspartic acid is in the form of a sodium salt.
